# EUROPEAN PATENT APPLICATION

(11) **EP 2 626 069 A1**
(43) Date of publication of application: **14.08.2013**
(21) Application number: 11830704.0
(22) Date of filing: 05.10.2011
(51) Int. Cl.: A61K 31/22, A61P 7/10

(54) **PROPHYLACTIC AND/OR THERAPEUTIC AGENT AGAINST LYMPHEDEMA**

(30) Priority: 06.10.2010 JP 2010226603
(71) Applicant: The University of Tokyo, Bunkyo-Ku Tokyo 113-8654 (JP); Kowa Company, Ltd., Nagoya-shi, Aichi-ken 460-8625 (JP)
(72) Inventor: MANABE, Ichiro, Tokyo 113-8654 (JP); NAGAI, Ryozo, Tokyo 113-8654 (JP); OGATA, Fusa, Tokyo 113-8654 (JP)
(74) Representative: Blodig, Wolfgang
(86) International application number: PCT/JP2011/072990
(87) International publication number: WO 2012/046772

(57) **Abstract**

Provided is a new prophylactic and/or therapeutic agent for lymphedema, for which until now there has been no effective means of treatment, and the prophylactic and/or therapeutic agent for lymphedema comprises, as an active ingredient, a compound represented by the following formula (1), a lactone derivative thereof, or a salt of the compound or lactone derivative: 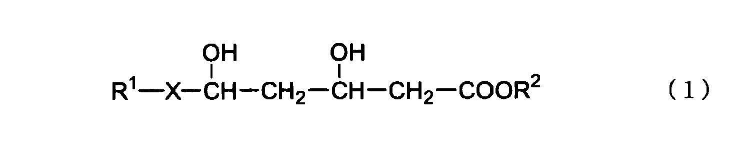 wherein R¹ represents an organic group, X represents -CH₂CH₂- or -CH=CH-, and R² represents a hydrogen atom or an alkyl group.

## Description

### [Technical Field]

The present invention relates to a prophylactic and/or therapeutic agent for lymphedema, for which until now there has been no effective therapy.

### [Background Art]

Lymphedema is a severe disease associated with hypertrophy and functional impairment of the extremities, repeated cellulitis and lymphorrhea, and the like caused by dysfunction of lymphatic vessels and retention of lymph fluid, which greatly reduces QOL in patients. Despite the fact that many patients experience difficulties even in their daily lives, there is no effective prophylactic or therapeutic method available. At present, lymphedema is managed by temporary alleviation of oedema by massaging, compression therapy using elastic stockings, and the like. The current situation is that despite the fact that lymphedema is such a severe disease as described above, a clear diagnostic standard has not yet been established, and further, because even healthcare workers are less aware of lymphedema as a disease which greatly impairs QOL in patients, there is no clear data of the number of patients.

In Japan, the vast majority of cases of lymphedema are secondary lymphedema, which develops after lymphadenectomy performed in a surgical operation or gynecologic surgery. These are not just acute diseases caused by postoperative lymphatic obstruction or removal of lymphatic vessels, but most of such cases develop three to five years after surgery. The mechanism of disease development remains totally unknown, and there is no effective prophylactic method. However, it is reported that 10 to 30% of patients after breast cancer surgery and 12 to 40% of patients after uterine cancer surgery develop lymphedema, and it is estimated that there are 30,000 to 50,000 patients with upper-extremity lymphedema and 50,000 to 70,000 patients with lower-extremity lymphedema in Japan.

As a means of treatment for lymphedema, increasing nitric oxide (NO) in lymphatic vessels has been proposed (Patent Literature 1). This literature describes that based on the observation that lymphatic flow was decreased by suppression of the production of NO, conversely, it might be possible to increase lymphatic flow by increasing NO, listing statins as examples of drugs for increasing NO. Also, Patent Literature 2 describes an improvement in lymphedema by the administration of VEGF-C, describing the possibility of treatment of lymphedema by anti-inflammatory agents.

### [Citation List]

### [Patent Literature]

[Patent Literature 1] WO2005/107461
[Patent Literature 2] US2008/0051644 Al

### [Summary of Invention]

### [Technical Problem]

However, it is well known that lymphedema is totally different from ordinary oedema caused by inflammation, and thus is incurable by anti-inflammatory agents such as non-steroidal anti-inflammatory drugs.
Accordingly, an object of the present invention is to provide an effective, novel prophylactic and/or therapeutic agent for lymphedema, for which until now there has been no effective therapy.

### [Solution to Problem]

In light of the above, based on the observation that lymphedema often develops after lymphadenectomy performed in a surgical operation or gynecologic surgery, the present inventors produced a lymphedema model by lymphatic vessel ligation. As a result, they found that ligation at multiple sites in lymphatic vessels connecting inguinal lymph nodes and axillary lymph nodes in a mouse caused oedema similar to lymphedema in humans and leakage of lymph fluid into tissues with good reproducibility. Using this model, the present inventors studied the actions of statins, which are HMG-CoA reductase inhibitors, on lymphedema. As a result, they found that statins had excellent inhibitory effects on lymphedema. Further, they studied the actions of statins on lymphedema using an endothelial nitric oxide synthase (eNOS)-deficient mouse to examine the relationship between lymphedema and NO. As a result, they found that the inhibitory effects of statins on lymphedema were unrelated to the production of NO, thereby completing the present invention.

That is, the present invention relates to the following [1] to [16].
[1] A prophylactic and/or therapeutic agent for lymphedema comprising, as an active ingredient, a compound represented by the following formula (1) or a lactone derivative thereof, or a salt of the compound or lactone derivative:

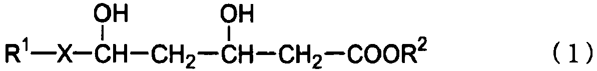

wherein R¹ represents an organic group, X represents -CH₂CH₂- or -CH=CH-, and R² represents a hydrogen atom or an alkyl group.
[2] The prophylactic and/or therapeutic agent for lymphedema according to [1], wherein R¹ is an indolyl group, an indenyl group, a pyridyl group, a pyrrolopyridyl group, a pyrazolopyridyl group, a thienopyridyl group, a pyrimidyl group, a pyrazolyl group, a pyrrolyl group, an imidazolyl group, an indolizinyl group, a quinolyl group, a naphthyl group, a hexahydronaphthyl group, a cyclohexyl group, a phenylsilylphenyl group, a phenylthienyl group, or a phenylfuryl group, all of which optionally have a substituent.
[3] The prophylactic and/or therapeutic agent for lymphedema according to [1], wherein the active ingredient is lovastatin, simvastatin, pravastatin, fluvastatin, cerivastatin, atorvastatin, rosuvastatin, mevastatin, pitavastatin, or a salt of any of the above.
[4] The prophylactic and/or therapeutic agent for lymphedema according to [1], wherein the active ingredient is pravastatin, atorvastatin, pitavastatin, or a salt of any of the above.
[5] A compound represented by the following formula (1) or a lactone derivative thereof, or a salt of the compound or lactone derivative for prophylaxis and/or treatment of lymphedema:

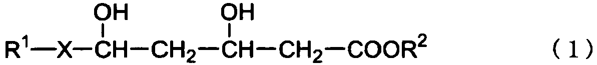

wherein R¹ represents an organic group, X represents -CH₂CH₂- or -CH=CH-, and R² represents a hydrogen atom or an alkyl group.
[6] The compound according to [5] or a lactone derivative thereof, or a salt of the compound or lactone derivative, wherein R¹ is an indolyl group, an indenyl group, a pyridyl group, a pyrrolopyridyl group, a pyrazolopyridyl group, a thienopyridyl group, a pyrimidyl group, a pyrazolyl group, a pyrrolyl group, an imidazolyl group, an indolizinyl group, a quinolyl group, a naphthyl group, a hexahydronaphthyl group, a cyclohexyl group, a phenylsilylphenyl group, a phenylthienyl group, or a phenylfuryl group, all of which optionally have a substituent.
[7] The compound according to [5] or a salt thereof, which is lovastatin, simvastatin, pravastatin, fluvastatin, cerivastatin, atorvastatin, rosuvastatin, mevastatin, pitavastatin, or a salt of any of the above.
[8] The compound according to [5] or a salt thereof, which is pravastatin, atorvastatin, pitavastatin, or a salt of any of the above.
[9] Use of a compound represented by the following formula (1) or a lactone derivative thereof, or a salt of the compound or lactone derivative for the production of a prophylactic and/or therapeutic agent for lymphedema:

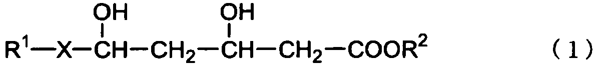

wherein R¹ represents an organic group, X represents -CH₂CH₂- or -CH=CH-, and R² represents a hydrogen atom or an alkyl group.
[10] The use according to [9], wherein R¹ is an indolyl group, an indenyl group, a pyridyl group, a pyrrolopyridyl group, a pyrazolopyridyl group, a thienopyridyl group, a pyrimidyl group, a pyrazolyl group, a pyrrolyl group, an imidazolyl group, an indolizinyl group, a quinolyl group, a naphthyl group, a hexahydronaphthyl group, a cyclohexyl group, a phenylsilylphenyl group, a phenylthienyl group, or a phenylfuryl group, all of which optionally have a substituent.
[11] The use according to [9], wherein the active ingredient is lovastatin, simvastatin, pravastatin, fluvastatin, cerivastatin, atorvastatin, rosuvastatin, mevastatin, pitavastatin, or a salt of any of the above.
[12] The use according to [9], wherein the active ingredient is pravastatin, atorvastatin, pitavastatin, or a salt of any of the above.
[13] A method for prophylaxis and/or treatment for lymphedema, characterized by administering a compound represented by the following formula (1) or a lactone derivative thereof, or a salt of the compound or lactone derivative:

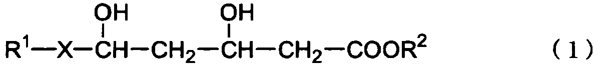

wherein R¹ represents an organic group, X represents -CH₂CH₂- or -CH=CH-, and R² represents a hydrogen atom or an alkyl group.
[14] The method according to [13], wherein R¹ is an indolyl group, an indenyl group, a pyridyl group, a pyrrolopyridyl group, a pyrazolopyridyl group, a thienopyridyl group, a pyrimidyl group, a pyrazolyl group, a pyrrolyl group, an imidazolyl group, an indolizinyl group, a quinolyl group, a naphthyl group, a hexahydronaphthyl group, a cyclohexyl group, a phenylsilylphenyl group, a phenylthienyl group, or a phenylfuryl group, all of which optionally have a substituent.
[15] The method according to [13], wherein the active ingredient is lovastatin, simvastatin, pravastatin, fluvastatin, cerivastatin, atorvastatin, rosuvastatin, mevastatin, pitavastatin, or a salt of any of the above.
[16] The method according to [13], wherein the active ingredient is pravastatin, atorvastatin, pitavastatin, or a salt of any of the above.

### [Effects of Invention]

According to the present invention, lymphedema, for which until now there has been no effective therapy, can be markedly inhibited; therefore, QOL in patients after a surgical operation or gynecologic surgery can be markedly improved.

### [Brief Description of Drawings]

[Figure 1] Figure 1 shows the action of pitavastatin on lymphedema (the thickness of the skin) in the wild-type and eNOS-deficient mice.
[Figure 2] Figure 2 shows the actions of pitavastatin, atorvastatin, and pravastatin on lymphedema (the thickness of the skin).
[Figure 3] Figure 3 shows the action of pitavastatin on leakage of lymph fluid in lymphedema.

### [Description of Embodiments]

The organic group represented by R¹ in the compound represented by the formula (1) is preferably an organic group having a cyclic structure which may have a substituent.
Examples of the organic group having a cyclic structure include an indolyl group, an indenyl group, a pyridyl group, a pyrrolopyridyl group, a pyrazolopyridyl group, a thienopyridyl group, a pyrimidyl group, a pyrazolyl group, a pyrrolyl group, an imidazolyl group, an indolizinyl group, a quinolyl group, a naphthyl group, a hexahydronaphthyl group, a cyclohexyl group, a phenylsilylphenyl group, a phenylthienyl group, and a phenylfuryl group, of which a hexahydronaphthyl group, an indolyl group, a pyridyl group, a pyrimidyl group, a pyrrolyl group, and a quinolyl group are particularly preferable.

Examples of a substituent which may be present in the above organic groups having a cyclic structure include a hydroxy group, a linear or branched, or cyclic alkyl group, an alkyloxyalkyl group, an alkylcarbonyloxy group, an alkyl-substituted amino group, a substituted alkylsulfonylamino group, a substituted phenylsulfonylamino group, and a carbamoyl group which may be substituted with one or two alkyl, phenyl, and the like, a halophenyl group, an alkylphenyl group, an alkoxyphenyl group, a phenyl group, and an oxo group.

Among these substituents which may be present in the above organic groups having a cyclic structure, a linear, branched, or cyclic C₁₋₆ alkyl group, a C₂₋₇ alkyloxyalkyl group, a C₁₋₄ acyloxy group, a C₁₋₄ alkyl-substituted amino group, a C₁₋₄ alkyl-substituted C₁₋₄ alkylsulfonylamino group, a C₁₋₄ alkyl-substituted phenylsulfonylamino group, a C₁₋₄ alkyl-substituted carbamoyl group, a phenyl-substituted carbamoyl group, a fluorophenyl group, a bromophenyl group, an iodophenyl group, a methylphenyl group, an ethylphenyl group, a methoxyphenyl group, an ethoxyphenyl group, and a phenyl group are preferable, of which an isopropyl group, a cyclopropyl group, and a p-fluorophenyl group are particularly preferable.

Examples of the alkyl group represented by R² include a linear, branched, or cyclic alkyl group having 1 to 6 carbon atoms.

A lactone derivative is obtained by subjecting a corresponding compound represented by the formula (1) to a routine lactonization method under, for example, acidic conditions.

A salt of the compound represented by the formula (1) or a lactone derivative thereof is a physiologically acceptable salt. Examples of the salt include an alkali metal salt such as a sodium salt and a potassium salt, an alkaline earth metal salt such as a calcium salt and a magnesium salt, and an organic amine salt such as a phenethylamine salt, and an ammonium salt, of which a sodium salt and a calcium salt are more preferable.

These compounds are described in, for example, U.S. Patent No. 4,739,073 and European Patent Application Publication No. 114,027; European Patent Application Publication No. 367,895; U.S. Patent Nos. 5,001,255, 4,613,610, 4,851,427, 4,755,606, 4,808,607, 4,751,235, 4,939,159, 4,822,799, 4,804,679, 4,876,280, 4,829,081, 4,927,851, and 4,588,715; and F. G. Kathawala, Medical Research Reviews, 11, 121 to 146 (1991), European Patent Application Publication No. 304,063; European Patent Application Publication No. 330,057, U.S. Patent Nos. 5,026,708, 4,868,185; European Patent Application Publication No. 324,347; European Patent Application Publication No. 300,278; U.S. Patent Nos. 5,013,749, 5,872,130, 5,856,336, U.S. Patent No. 4,231,938, U.S. Patent No. 4,444,784, U.S. Patent No. 4,346,227, U.S. Patent No. 5,354,772, U.S. Patent No. 5,273,995, U.S. Patent No. 5,177,080, U.S. Patent No. 3,983,140, JP-B-2,648,897, U.S. Patent No. 5,260,440, Bioorganic & Medicinal Chemistry, 5, 437, (1977), JP-B-2,569,746, European Patent No. 304,063, and U.S. Patent No. 5,856,336.

Examples of the active ingredient of the prophylactic and/or therapeutic agent for lymphedema according to the present invention include lovastatin (U.S. Patent No. 4,231,938: (+)-(1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthyl(S)-2-methylbutyrate), simvastatin (U.S. Patent No. 4,444,784: (+)-(1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthyl 2,2-dimethylbutanoate), pravastatin (U.S. Patent No. 4,346,227: (+)-(3R,5R)-3,5-dihydroxy-7-[(1S,2S,6S,8S,8aR)-6-hydroxy-2-methyl-8-[(S)-2-methylbutyryloxy]-1,2,6,7,8,8a-hexahydro-1-naphthyl]heptanoic acid), fluvastatin (U.S. Patent No. 5,354,772: (3RS,5SR,6E)-7-[3-(4-fluorophenyl)-1-(1-methylethyl)-1H-indol-2-yl]-3,5-dihydroxy-6-heptenoic acid), atorvastatin (U.S. Patent No. 5,273,995: (3R,5R)-7-[2-(4-fluorophenyl)-5-isopropyl-3-phenyl-4-phenylcarbamoyl-1H-pyrrol-1-yl]-3,5-dihydroxyheptanoic acid), cerivastatin (U.S. Patent No. 5,177,080: (3R,5S)-erythro-(E)-7-[4-(4-fluorophenyl)-2,6-diisopropyl-5-methoxymethyl-pyridin-3-yl]-3,5-dihydroxy-6-heptenoic acid), mevastatin (U.S. Patent No. 3,983,140: (+)-(1S,3R,7S,8S,8aR)-1,2,3,7,8,8a-hexahydro-7-methyl-8-[2-[(2R,4R)-tetrahydro-4-hydroxy-6-oxo-2H-pyran-2-yl]ethyl]-1-naphthyl(S)-2-methylbutyrate), rosuvastatin (U.S. Patent No. 5,260,440, JP-B-2,648,897: 7-[4-(4-fluorophenyl)-6-isopropyl-2-(N-methyl-N-methanesulfonylaminopyrimidine)-5-yl]-(3R,5S)-dihydroxy-(E)-6-heptenoic acid), pitavastatin (U.S. Patent No. 5,856,336, JP-B-2,569,746: (3R,5S,6E)-7-[2-cyclopropyl-4-(4-fluorophenyl)-3-quinolyl]-3,5-dihydroxy-6-heptenoic acid, and a salt of any of the above, of which pravastatin or a salt thereof, pitavastatin or a salt thereof, and atorvastatin or a salt thereof are particularly preferable.

The compound represented by the formula (1), a lactone derivative thereof, or a salt of the compound or lactone derivative (hereinbelow, may also be referred to as statins) markedly inhibits lymphedema, and also inhibits leakage of lymph fluid into tissues, and thus is useful as a prophylactic and/or therapeutic agent for lymphedema. Also, the statins of the present invention exerted inhibitory actions on lymphedema in a non-eNOS-expressing model as well, revealing that the above action is exerted independently of the NO production action of statins. A more prominent inhibitory action of statins on lymphedema is achieved by the administration before surgery, which is presumed to cause the development of lymphedema, or before the post-operative development of lymphedema. Further, considering that statins have already been widely used as a therapeutic drug for hypercholesterolemia, there is no problem regarding safety.

Examples of the dosage form of the statins of the present invention include oral administration in the form of, for example, a tablet, a capsule, a granule, a powder, and a syrup, and parenteral administration in the form of, for example, an intravenous injection, an intramuscular injection, a suppository, an inhalant, a percutaneous absorption agent, an eye drop, and a nasal drop. Of these, oral administration is preferable.
Also, in order to prepare medicinal preparations of various dosage forms such as ones mentioned above, the above active ingredients can be used alone or in an appropriate combination with one or more other pharmaceutically acceptable excipients, binders, expanders, disintegrants, surfactants, lubricants, dispersants, buffers, preservatives, flavors, fragrances, coating agents, carriers, diluents, and the like.

Among the aforementioned dosage forms, oral administration is preferable. In preparing an orally administered preparation, it is preferable to adjust pH (JP-A-2-6406, JP-B-2,774,037, WO97/23200, and the like) in consideration of the stability of the active ingredient.

Although the dose of the above drugs varies according to the body weight, age, sex, and symptoms of the patient, normally for an adult, the drugs are orally administered in an amount of preferably 0.01 to 1000 mg, more preferably 0.1 to 100 mg, and particularly preferably 1 to 50 mg in terms of the compound represented by the formula (1) once or in several divided doses a day.

Considering that most cases of lymphedema develop after several years post-surgery, the timing of administration of statins is desirably determined according to either a means of prophylactic administration before the post-operative development of lymphedema or a means of administration after the development of lymphedema for the improvement of the symptoms, of which prophylactic administration before the post-operative development of lymphedema is preferable. In that case, the dose is preferably determined as described above.

### [Examples]

Hereinbelow, the present invention will be described in detail with reference to the Examples; however, the present invention is not limited to these Examples.

### Examples

### (Experimental method)

### (1) Production of a mouse model of abdominal wall lymphedema

A male C57BL/6J mouse and a male eNOS-deficient mouse having a genetic background of C57BL/6J, both of which are eight weeks old at the time of surgery, each receives an intraperitoneal injection of Nembutal at 60 mg/kg, and further receives a subcutaneous injection of 1% Evans blue for identification of lymph nodes and lymphatic vessels in the dorsum of the right foot, the back of the hand, and the buttocks in an amount of 0.05 mL at each site. The abdominal hair was removed and the abdominal skin is incised in an open-door fashion so as to identify the inguinal lymph nodes, axillary lymph nodes, and lymphatic vessels connecting these lymph nodes in the right abdominal wall. The lymphatic vessels are ligated at two to three sites near the axillary lymph nodes, followed by excision of the axillary lymph nodes. Subsequently, the skin is sutured, whereby the surgery is completed. On the seventh day after the surgery, the abdominal skin is once again incised in an open-door fashion to collect the abdominal wall tissues.

### (2) Method of administering statins

The mice were orally administered with the statins dissolved in distilled water (250 µl) from three days before to seven days after the surgery once a day. To the mice in the control group distilled water was given in a similar manner.
Experiment 1) C57BL/6J Mice (n=4) and eNOS-deficient mice having a genetic background of C57BL/6J (n=4) were administered with pitavastatin at 10 mg/kg/day. Experiment 2) C57BL/6J Mice were administered with pitavastatin at 1 mg/kg/day and atorvastatin at 2.5 mg/kg/day (n=4 for each statin).
Experiment 3) C57BL/6J Mice were administered with pitavastatin at 1 mg/kg/day, atorvastatin at 1 mg/kg/day, and pravastatin at 1 mg/kg/day (n = 3, 4, and 3 for each statin).

### (Evaluation of drug efficacy)

### 1) Visual examination

On the seventh and fourteenth days after the surgery, 0.05 µl of 1% Evans blue was locally injected into the inguinal lymph nodes for lymphangiography, whereby leakage of lymph fluid and lymphatic flow were evaluated.

### 2) Evaluation of oedema by the thickness of the subcutaneous tissues

Methanol-fixed paraffin-embedded tissue sections were stained with hematoxylin, and the thickness of the skin section prepared from the upper inguinal area, which is the lowermost part of a mouse, was measured. Anatomically, interstitial fluid tends to be accumulated in this area.

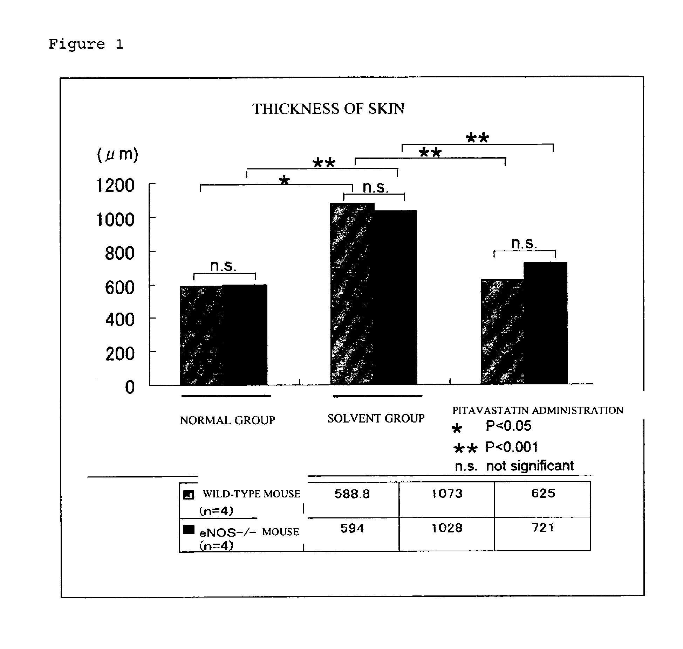

The thickness of the skin section prepared from the upper inguinal area after seven days in the normal group, solvent (water)-administered group, and pitavastatin-administered group is shown in Figure 1 (the results of Experiment 1). In Figure 1, the gray and black bars each represent the results obtained from the wild-type mouse and eNOS-deficient mouse, respectively. Lymphedema was markedly inhibited by administration of pitavastatin in the wild-type mouse as well as in the eNOS-deficient mouse. These results reveal that statins markedly inhibit lymphedema, regardless of the presence or absence of the expression of eNOS. That is, the inhibitory actions of statins on lymphedema are clearly unrelated to the production of NO.

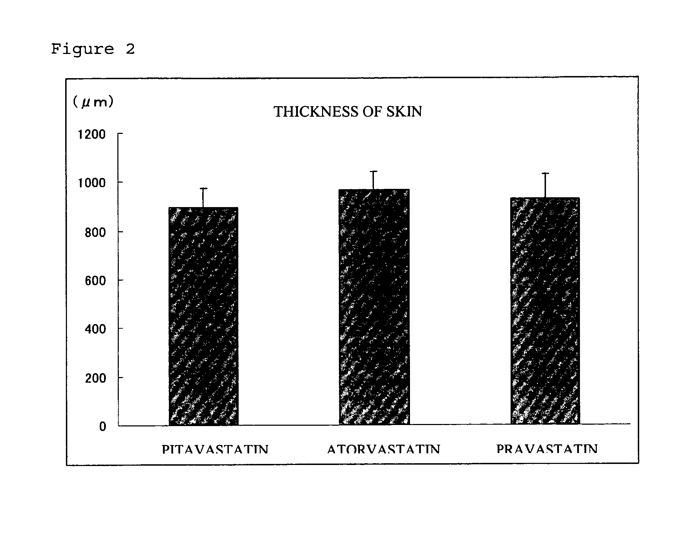

Also, the thickness of the skin after administration of pitavastatin, atorvastatin, and pravastatin (after seven days) is shown in Figure 2 (the combined results of Experiments 2 and 3 are shown). It is understood that similarly to pitavastatin, atorvastatin and pravastatin also markedly inhibit lymphedema.

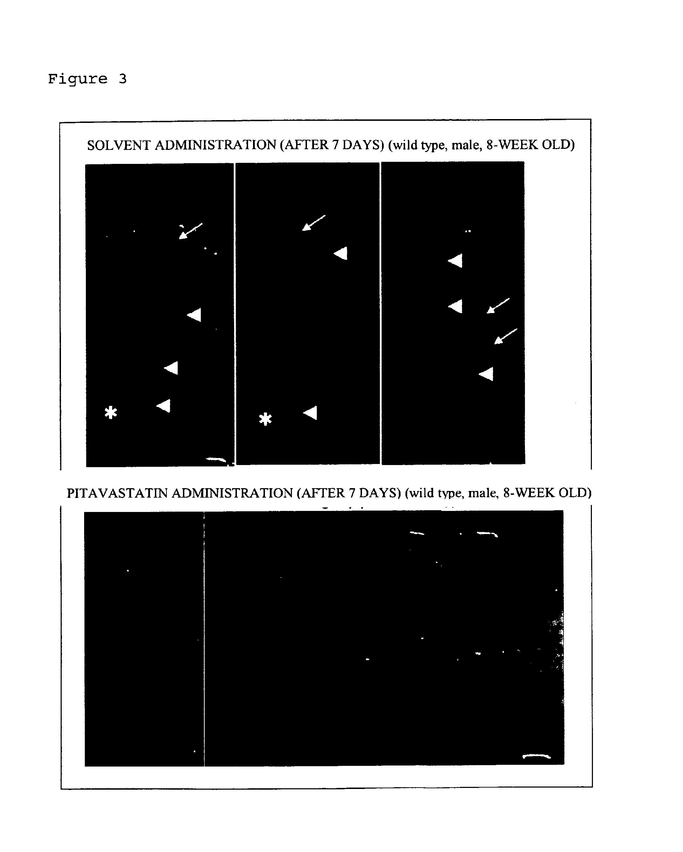

Each statin was administered by the administration method employed in Experiment 3) and the leakage of lymph fluid and lymphatic flow were evaluated by lymphangiography of the lymph node tissues. As a result, notable leakage of lymph fluid was found in the solvent-administered group after seven days as well as after 14 days. In contrast, in the pitavastatin-administered group, leakage of lymph fluid was markedly inhibited (Figure 3). Also, similar results were observed in the atorvastatin-administered group and pravastatin-administered group.

## Claims

1. A prophylactic and/or therapeutic agent for lymphedema comprising, as an active ingredient, a compound represented by the following formula (1) or a lactone derivative thereof, or a salt of the compound or lactone derivative: 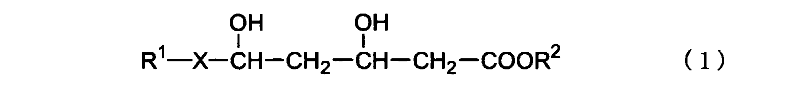 wherein R¹ represents an organic group, X represents -CH₂CH₂- or -CH=CH-, and R² represents a hydrogen atom or an alkyl group.

2. The prophylactic and/or therapeutic agent for lymphedema according to claim 1, wherein R¹ is an indolyl group, an indenyl group, a pyridyl group, a pyrrolopyridyl group, a pyrazolopyridyl group, a thienopyridyl group, a pyrimidyl group, a pyrazolyl group, a pyrrolyl group, an imidazolyl group, an indolizinyl group, a quinolyl group, a naphthyl group, a hexahydronaphthyl group, a cyclohexyl group, a phenylsilylphenyl group, a phenylthienyl group, or a phenylfuryl group, all of which optionally have a substituent.

3. The prophylactic and/or therapeutic agent for lymphedema according to claim 1, wherein the active ingredient is lovastatin, simvastatin, pravastatin, fluvastatin, cerivastatin, atorvastatin, rosuvastatin, mevastatin, pitavastatin, or a salt of any of the above.

4. The prophylactic and/or therapeutic agent for lymphedema according to claim 1, wherein the active ingredient is pravastatin, atorvastatin, pitavastatin, or a salt of any of the above.

5. A compound represented by the following formula (1) or a lactone derivative thereof, or a salt of the compound or lactone derivative for prophylaxis and/or treatment of lymphedema: 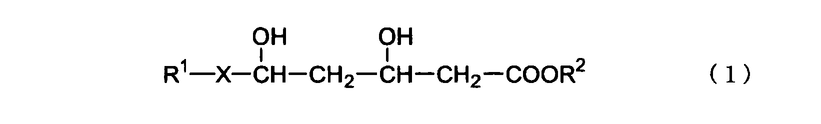 wherein R¹ represents an organic group, X represents -CH₂CH₂- or -CH=CH-, and R² represents a hydrogen atom or an alkyl group.

6. The compound according to claim 5 or a lactone derivative thereof, or a salt of the compound or lactone derivative, wherein R¹ is an indolyl group, an indenyl group, a pyridyl group, a pyrrolopyridyl group, a pyrazolopyridyl group, a thienopyridyl group, a pyrimidyl group, a pyrazolyl group, a pyrrolyl group, an imidazolyl group, an indolizinyl group, a quinolyl group, a naphthyl group, a hexahydronaphthyl group, a cyclohexyl group, a phenylsilylphenyl group, a phenylthienyl group, or a phenylfuryl group, all of which optionally have a substituent.

7. The compound according to claim 5 or a salt thereof, which is lovastatin, simvastatin, pravastatin, fluvastatin, cerivastatin, atorvastatin, rosuvastatin, mevastatin, pitavastatin, or a salt of any of the above.

8. The compound according to claim 5 or a salt thereof, which is pravastatin, atorvastatin, pitavastatin, or a salt of any of the above.

9. Use of a compound represented by the following formula (1) or a lactone derivative thereof, or a salt of the compound or lactone derivative for the production of a prophylactic and/or therapeutic agent for lymphedema: 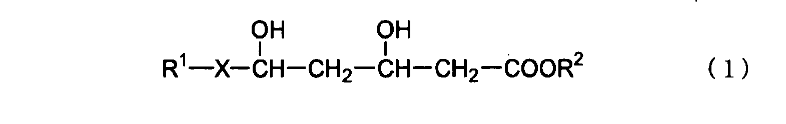 wherein R¹ represents an organic group, X represents -CH₂CH₂- or -CH=CH-, and R² represents a hydrogen atom or an alkyl group.

10. The use according to claim 9, wherein R¹ is an indolyl group, an indenyl group, a pyridyl group, a pyrrolopyridyl group, a pyrazolopyridyl group, a thienopyridyl group, a pyrimidyl group, a pyrazolyl group, a pyrrolyl group, an imidazolyl group, an indolizinyl group, a quinolyl group, a naphthyl group, a hexahydronaphthyl group, a cyclohexyl group, a phenylsilylphenyl group, a phenylthienyl group, or a phenylfuryl group, all of which optionally have a substituent.

11. The use according to claim 9, wherein the active ingredient is lovastatin, simvastatin, pravastatin, fluvastatin, cerivastatin, atorvastatin, rosuvastatin, mevastatin, pitavastatin, or a salt of any of the above.

12. The use according to claim 9, wherein the active ingredient is pravastatin, atorvastatin, pitavastatin, or a salt of any of the above.

13. A method for prophylaxis and/or treatment for lymphedema, comprising administering a compound represented by the following formula (1) or a lactone derivative thereof, or a salt of the compound or lactone derivative: 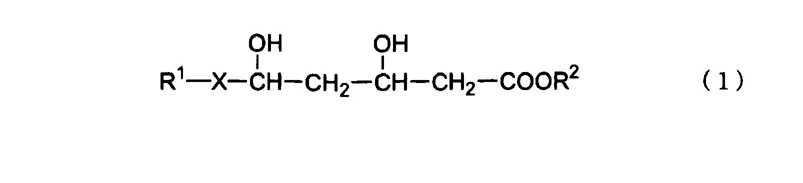 wherein R¹ represents an organic group, X represents -CH₂CH₂- or -CH=CH-, and R² represents a hydrogen atom or an alkyl group.

14. The method according to claim 13, wherein R¹ is an indolyl group, an indenyl group, a pyridyl group, a pyrrolopyridyl group, a pyrazolopyridyl group, a thienopyridyl group, a pyrimidyl group, a pyrazolyl group, a pyrrolyl group, an imidazolyl group, an indolizinyl group, a quinolyl group, a naphthyl group, a hexahydronaphthyl group, a cyclohexyl group, a phenylsilylphenyl group, a phenylthienyl group, or a phenylfuryl group, all of which optionally have a substituent.

15. The method according to claim 13, wherein the active ingredient is lovastatin, simvastatin, pravastatin, fluvastatin, cerivastatin, atorvastatin, rosuvastatin, mevastatin, pitavastatin, or a salt of any of the above.

16. The method according to claim 13, wherein the active ingredient is pravastatin, atorvastatin, pitavastatin, or a salt of any of the above.
